# EUROPEAN PATENT APPLICATION

(11) **EP 1 031 328 A1**
(43) Date of publication of application: **30.08.2000**
(21) Application number: 00103857.9
(22) Date of filing: 24.02.2000
(51) Int. Cl.: A61F 2/06

(54) **Bifurcated stent delivery balloon catheter**

(30) Priority: 26.02.1999 IT MI990392
(71) Applicant: AMS ITALIA S.r.l., 31050 Villorba (Treviso) (IT)
(72) Inventor: Morocutti, Giorgio, 33100 Udine (IT)
(74) Representative: Rapisardi, Mariacristina

(57) **Abstract**

Catheter for delivering a bifurcated stent with a two chambered balloon, a stent with a lateral opening and two guidewires to place the stent. One guidewire passes through the lateral opening in the stent to track the side branch of the stent.

## Description

The present invention relates to a new type of improved catheter for vascular procedures.

In order to treat arterial stenosis, it is known to dilate the arteries through an angioplasty procedure.

This operation is carried out by inserting a catheter into the artery and making a second small tube slide inside it, whereby the second tube is provided at an end thereof with a small balloon that is pushed along a metallic guidewire (intracoronary guidewire) of small dimensions up to the area of narrowing.

Once the area of narrowing area has been reached, the balloon is repeatedly inflated with a pressurized liquid, introduced through the small tube supporting it, so to press the plaques against the blood vessel walls and to re-establish the normal lumen dimensions. Furthermore, in order to avoid restenosis events, a stent is applied on the treated part of the blood vessel, whereby the stent consists of a small metallic frame, that supports the artery from inside, thus preventing it from narrowing.

Most lesions, which materialize in the generation of atherosclerotic plaques in the blood vessels, appears at the bifurcation level. In such case, the catheter action instead of pressing the plaques and therefore enlarging the lumen, tends to transfer them, making them slide towards that bifurcation branch, which is not filled by the balloon. Such an action, obviously, does not cure the stenosis, on the contrary it moves the stenosis from a branch to another.

On the other hand, the stent application is not always carried out in the right way, at the bifurcation level, by means of current angioplasty catheters. In fact, the stent obstructs mechanically the side blood flow towards the secondary branch of the bifurcation, and it also hinders the insertion of the guidewire therein.

Therefore, the use of the stent is critical at the bifurcation, because the balloon catheter, by which it is supported, does not allow to assess precisely the limit between the obstructed vessel wall and the starting point of the bifurcation. On the contrary, while the stent meshes, that lean against the obstructed vessel wall have to be as narrow as possible, those on the opening of the bifurcation have to be large enough not to hinder either the blood flow, or the possible insertion of a second small metall intracoronary guidewire.

Therefore, it is the main object of the present invention to provide a catheter that, in comparison with those currently used, makes it possible to treat effectively the stenosis also at the bifurcation level.

It is a further object of the invention to provide a catheter that allows to control the position of the stent with respect to the branches of the bifurcation, distinguishing between its obstructed wall and the opening of the lateral blood vessel.

These and other objects are achieved by means of a device such as claimed in claim 1. Other preferred embodiments of the device itself will become apparent from the remaining claims.

Differently from prior art devices, the significant advantage of the device according to the present invention lies in the possibily of mantaining the position of intracoronary guidewires in the two bifurcation branches so that it is possible to treat (even with stent) the vessels of the bifurcation itself.

Through the same device it is possible to select the position of the stent with respect to the bifurcation, so to allow the "precise" graft on the main branch, on the secondary one, or on both branches of the bifurcation itself.

The device of the invention further allows to select the orientation of the stent around its axis, so to direct its portion provided with larger meshes towards the opening of the lateral blood vessel; such large meshes are adapted to allow a free blood inflow.

Such device further allows to place stents with a slanted end (with slant angles ranging from 3o° to 60°) in such a way to respect the natural anatomy of the starting area of the collateral vessel (angled with respect to the main vessel).

These and other features and advantages will become more clear from the description hereinbelow of a preferred embodiment of the catheter of the present invention, shown as an example in the attached drawings. In such drawings:
figure 1 is a partial view of the balloon catheter according to the invention;
figure 2 shows a cross-section view of the catheter in figure 1 according to the finding;
figure 3 shows the catheter with the inflated balloon and stents mounted thereon;
figure 4 shows the catheter in figure 1 in cross-section II-II; figure 5 shows a cylindrical stent which can be used on the catheter according to the finding;
figure 6 shows the stent of figure 5 mounted on the balloon catheter according to finding;
figure 7 schematically shows the insertion of the balloon catheter having the stent mounted before being placed in a coronary bifurcation;
figure 8 shows the balloon catheter with the stent mounted, correctly placed in the coronary bifurcation;
figure 9 shows the catheter with inflated ballon and the extension of the stent in the coronary bifurcation;
figure 10 shows the stent placed after the deflation and the withdrawal of the balloon catheter;
figure 11 shows a different embodiment of the balloon catheter with the guidewire exiting sideways;

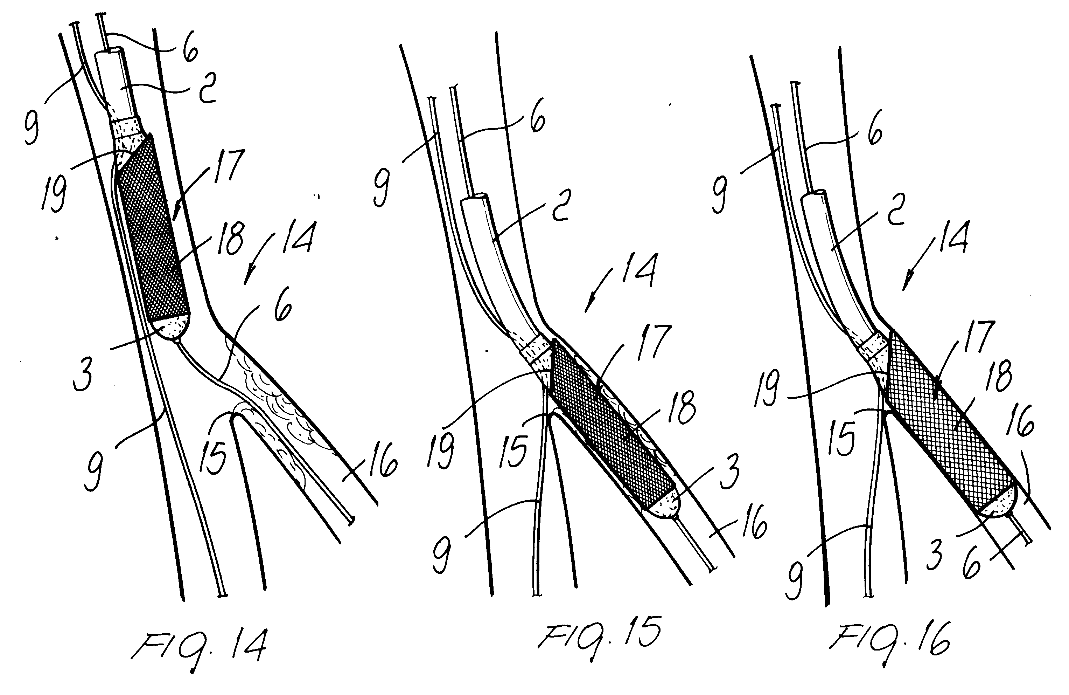

figure 12 is a perspective view of a different embodiment of the slanted stent;
figure 13 shows the slanted stent mounted on the catheter shown in figure 11;
figures from 14 to 16 show the positioning of the slanted stent im a coronary bifurcation;

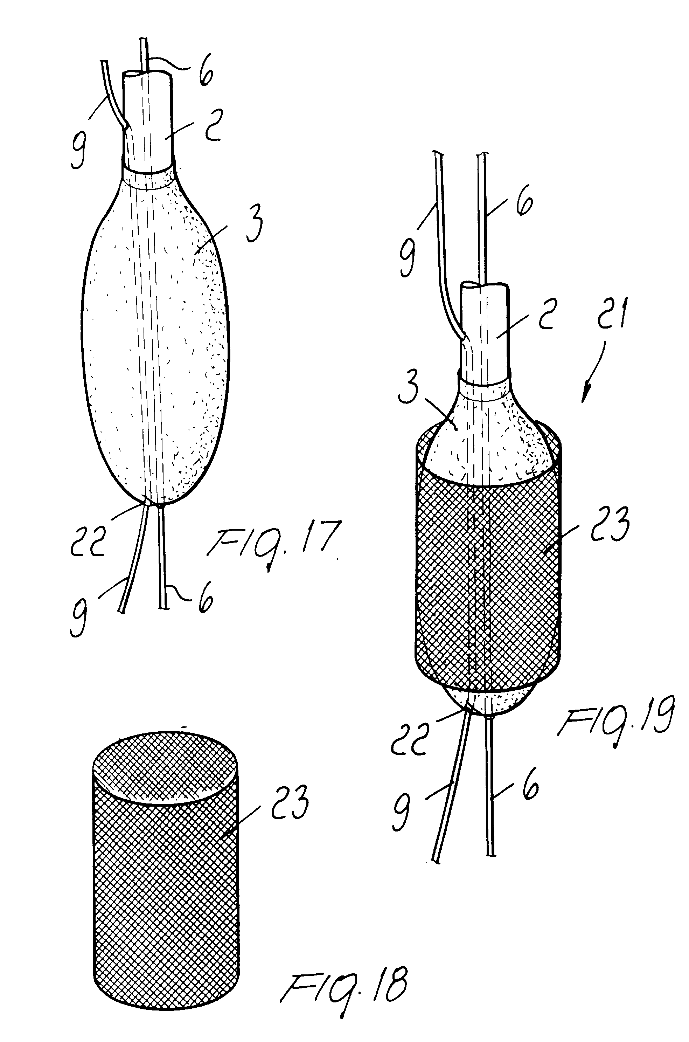

figures 17, 18 and 19 show the balloon catheter having a cylindrical stent without side opening mounted;

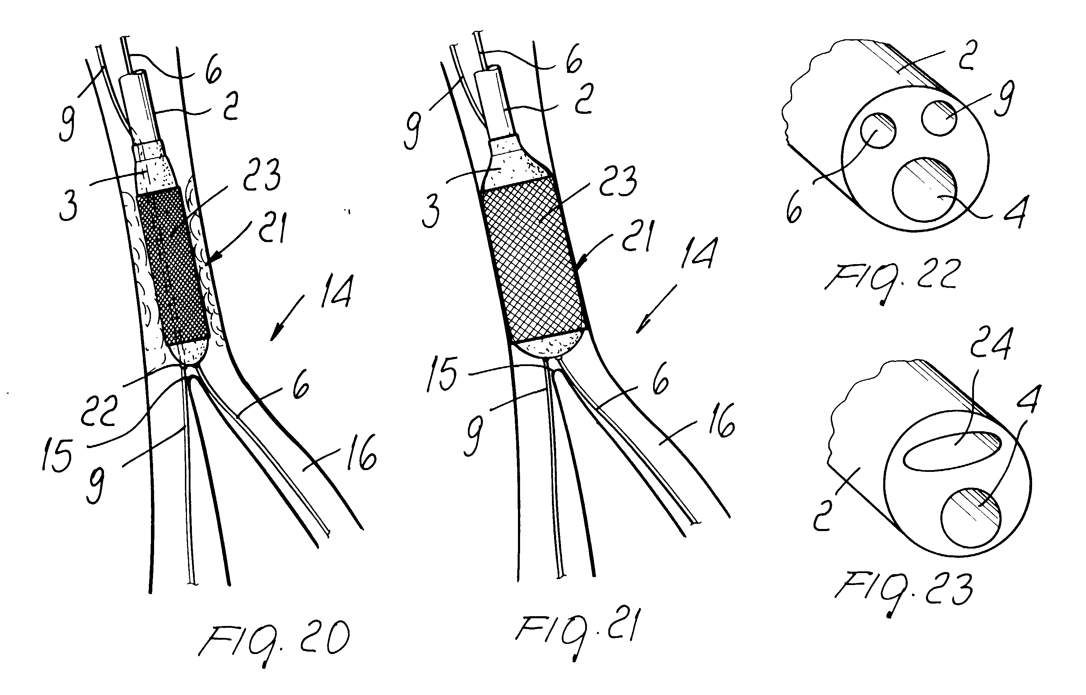

figures 20 and 21 show the positioning of the cylindrical stent of figure 18 before the coronary bifurcation; and
figures 22 and 23 are section views of two different embodiments of channels inside which the guidewires according to the finding are made pass.

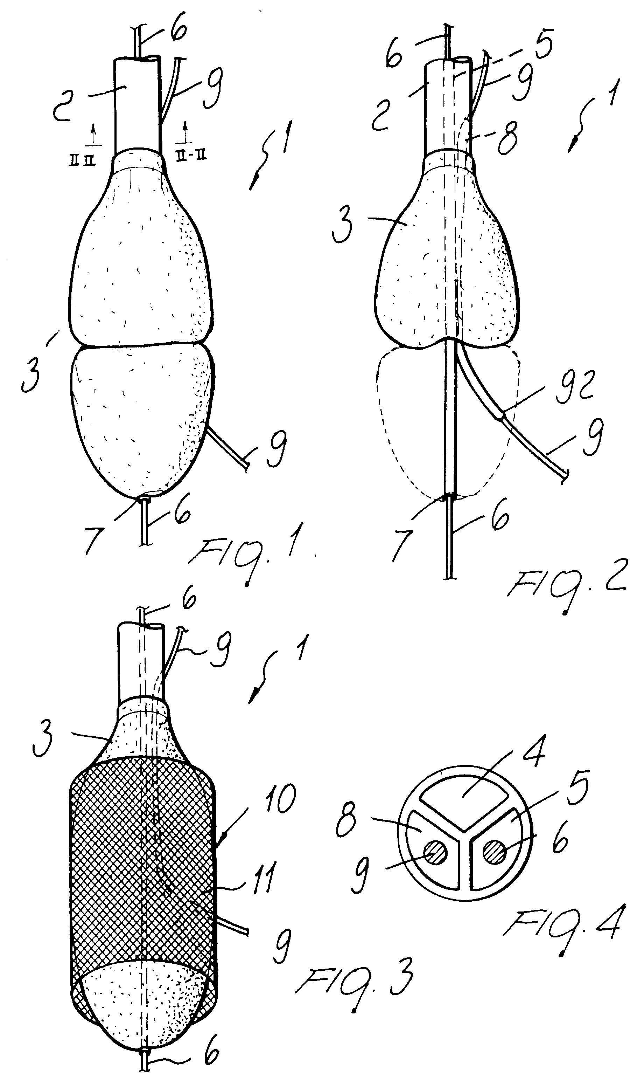

The catheter of the present invention illustrated in figure 1 is referenced to in its whole with 1. It is composed of a small tube 2 which carries at its end an inflatable or expandable balloon 3 made of elastomeric material. In particular it is the injection of a suitable contrast liquid in the channel 4 of tube 2, that produces such expansion.

Further on, the small tube 2 and the balloon 3 have a channel 5 for a first guidewire 6, preferably made of metallic material, that comes out from the tip 7 of the same balloon 3, as well as a channel 8 for a second guidewire 9, which is also preferably made of metal, which comes out sideways with respect to that balloon. In particular, this guidewire 9 is inserted into the channel 8 from an inlet 91 coming out from the hole or seat 92 provided in the balloon 3 sideways.

The catheter 1 can be equipped with the stent 10 illustrated in figure 3. In particular, the latter consists of a cylindrical cage 11 made of resistant material, preferably metal, which supports the vessel walls and is provided with a side opening 12. When applied on the device 1, the stent 10 is provided with an opening 12 oriented sideways with respect to the balloon 3 and the guidewire 9 that comes out from the latter (figure 4). The so arranged device reproduces the shape or form 13 of the saddle 15 of that bifurcation 14, where it will be applied (figure 4 and 5).

As shown in figue 5, the insertion of the device 1 into the vessel will be carried on, according to the direction of arrow F, until it engages the portion of guidewire 9 that comes out from the hole 92, with the saddle 15 of the bifurcation. As mentioned above, this engagement is made possible in particular by the reproduction on the device 1 itself, of the shape 13 of the bifurcation, shape corresponding to the side development of the guidewire 9 with respect to the balloon 3 and to the remaining guidewire 6.

In this embodiment, the stent 10 is applied at the bifurcation 14, with the opening 12 directed towards the secondary branch 16 of the vessel. By deflating the balloon 3, and withdrawing the guidewires 6 and 9, with a movement in the opposite direction of that for the insertion of the device 1, the stent 10 is kept in position in the bifurcation 14, with opening 12 directed in the sense of the side blood flow towards the secondary vessel 16.

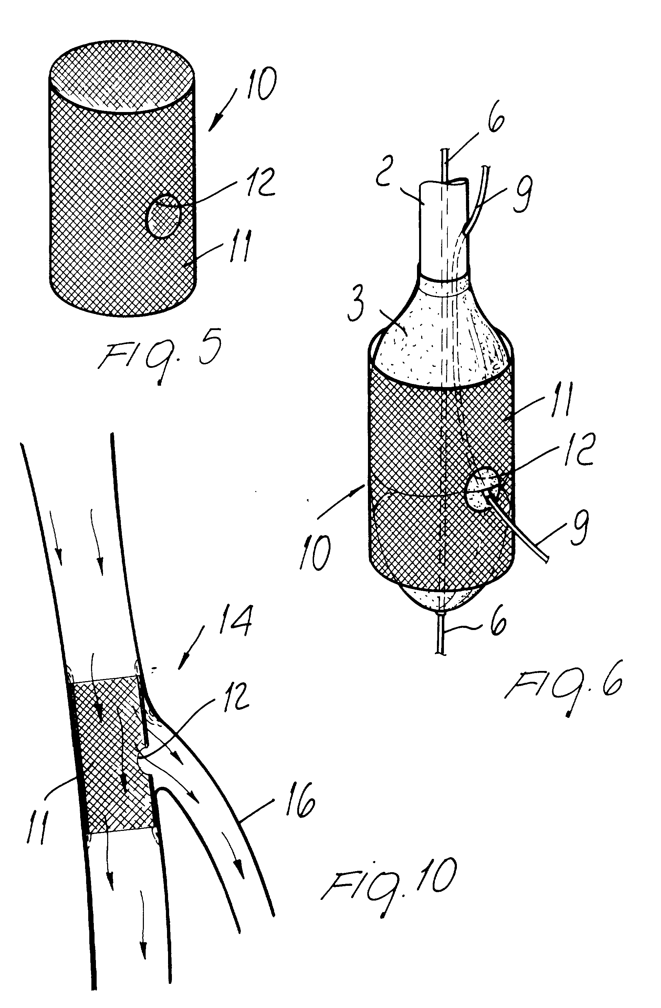

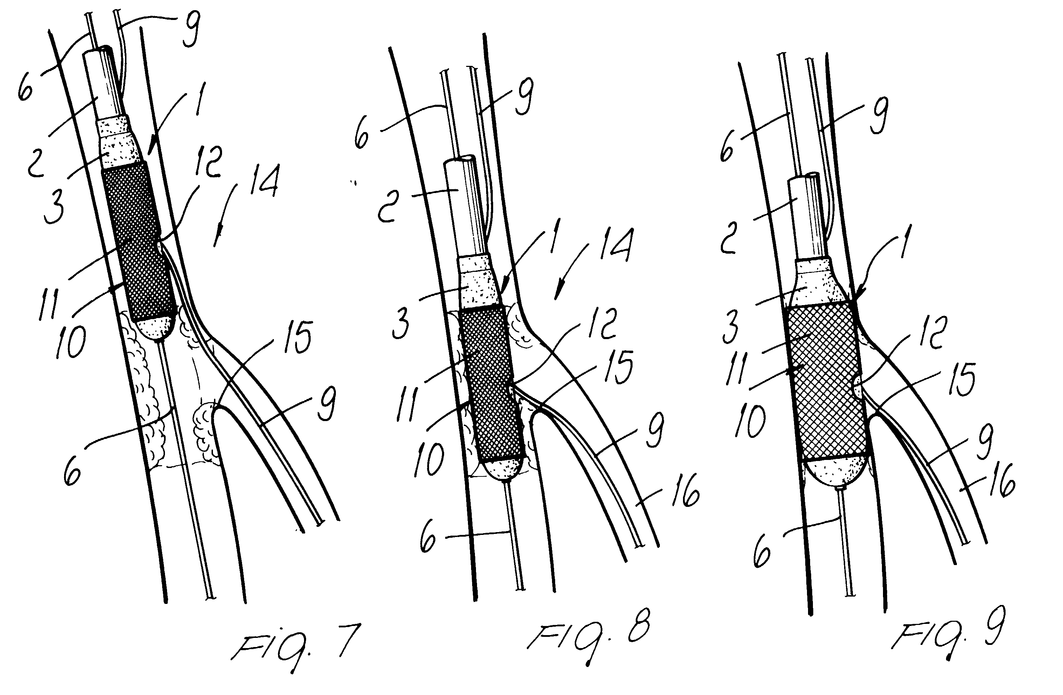

In the embodiment illustrated in figure 6, the catheter of the invention is referred to with 17. According to this embodiment, the second guidewire 9 comes out of the balloon 3 at a seat 20 provided in proximal or back position in the balloon itself. As it is also shown in figure 8, the catheter 17 is adapted to support a stent 18 with a 19° to 45° bent or slanted bevel(figure 7). With respect to the application of figure 4, in this case the guidewires 6 and 9 reproduce the shape 13 of the bifurcation, moved from a medial position to a proximal one according to the saddle 15 of the bifurcation itself (figure 8 and 9).

The insertion of the device 17, in the direction of the arrow F of figure 9, in the bifurcation 14 is carried on until the stent is inserted in the branch 18 and it interferes with the saddle 15 of the bifurcation 14, of the portion of the guidewire 9 that comes out from the seat 20, sideways with respect to the balloon 3 (figure 9).

It shall in particular be noted that the bevelled configuration of the end 19 of the stent itself avoids providing a sharp edge or projecting inside the main blood vessels, that guarantees the free blood circulation and the absence of dangerous turbolences.

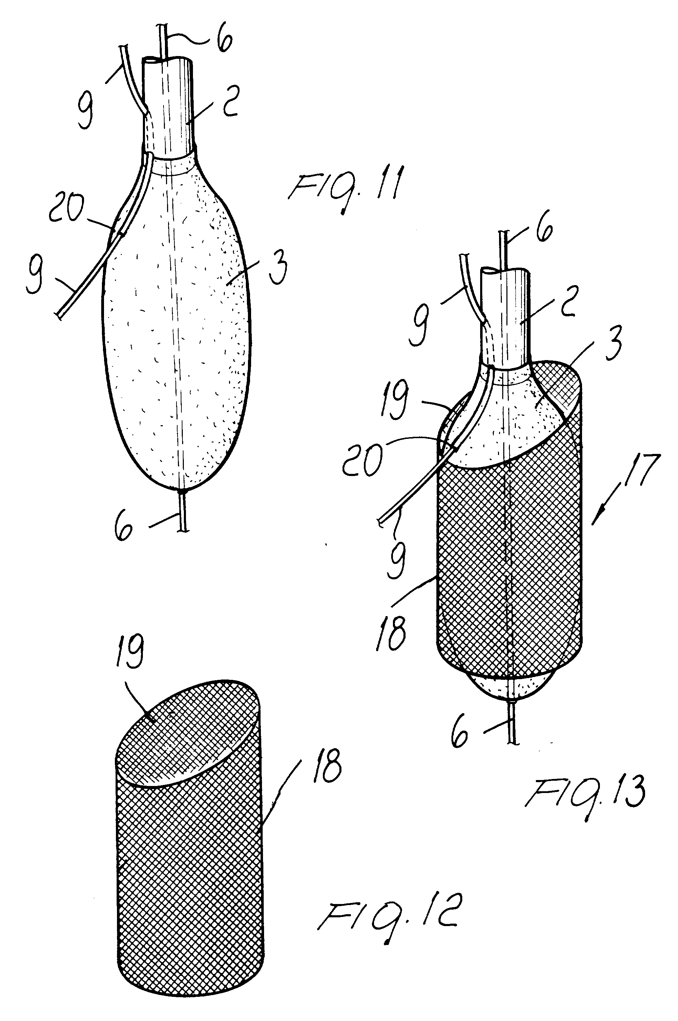

In the embodiment illustrated in figure 10, the catheter of the invention is referred to with 21. According to this embodiment, the second guidewire 9 comes out from the balloon 3 in a distal or front position 22 with respect to the balloon itself (figure 10).

Moreover, in this embodiment, the catheter 21 is provided with a substantially tube-shaped stent 23.

Inserting the catheter 21, in the direction of the arrow F of figure 11 in the main branch of a bifurcation 14, at a certain point there will be an interference with the portion of guidewire 9 that comes out of the seat 22 of the catheter itself, with the saddle 15 of the bifurcation itself. In this case, as the shape 13 of the latter has been reproduced on the catheter 21 in a distal or front position with respect to the balloon, the stent 23 will be applied upstream of the saddle 15 of the bifurcation, so to protect the main branch of the coronary against relapsing stenosis events.

Modifications to the above disclosed and illustrated invention may be implemented, in order to carry out alternative embodiments, that however fall within the scope of the attached claims. Thus, for example, provided that the two-guidewire system is present, the configuration and the design of the balloon could be different.

In particular, in the case of the catheter with side opening for the guidewire, rather than as an integral item, as shown in figure 1, the balloon can be designed in form of two separated chambers at the side opening for the guidewire, as it is for example illustrated in figures 2 and 11.

As illustrated in figures 22 and 23, the seats of the guidewires 6 and 9 could be also designed in form of respective circular section channels, that hinder the overlapping of the guidewires during the insertion into the blood vessel and allow to maintain its correct positioning.

Instead of the abovementioned channels, a single channel 24 could be provided (figure 23) which is flattened enough or provided with an ellipsoidal cross-section, adapted to achieve the same object. As alternative to what disclosed and illustrated, this device could be also equipped with a number of guidewires higher than two, and the used stents could have any shape or design.

## Claims

1. Improved catheter for vascular procedures of the type comprising a small tube (2) provided at the end thereof with an balloon expandable by the action of a contrast liquid supplied through said tube (2), characterized in that it is provided with means adapted to reproduce on said balloon (3) the shape (13) of the coronary bifurcation (14).

2. Catheter according to claim 1, characterised in that said means are composed of a system including at least two guidewires (6, 9), of which a first guidewire (6) is orientated in axial direction with respect to the longitudinal extension of the balloon (3) and at least another guidewire (9) is provided sideways with respect to the first one, so to form on said balloon a lateral bifurcation that reproduces together with the guidewire (6) the shape (13) of said bifurcation (14).

3. Catheter according to claims 1 and 2, characterized in that said small tube (2) is provided not only with a channel (4) for supplying said contrast liquid to the balloon (3), but also with respective channels (5, 8) for the access of the guidewires (6, 9) to said balloon.

4. Catheter according to one or more of the preceding claims, characterised in that said balloon (3) is provided with at least an opening or side seat (92) for the exit from said balloon of said at least another guidewire (9) in branching or lateral arrangement with respect to the first guidewire (6).

5. Cathether according to one or more of the preceding claims, characterised in that said balloon (3) is provided with a stent (10) composed of a cage (11) of resistant material provided with a side opening (12) at the seat (92) for the exit of said side guidewire (9) of the balloon (3).

6. Catheter according to claims 4 or 5, characterized in that the portion of the side guidewire (9) that comes out from said seat (92) forms the abutment for the catheter (1) positioning onto the saddle (15) of the bifurcation (14), whereby, when the catheter (1) is so positioned, the opening (12) of the stent (10) is directed towards the side branch (16) of the bifurcation (14).

7. Catheter according to one or more of the claims 1 to 3, characterized in that said balloon (3) is provided with at least one opening or seat (20) for the exit in proximal position with respect to said balloon of said at least another guidewire (9) in branching or lateral arrangement with respect to the first guidewire (6).

8. Catheter according to claim 7, characterised in that said balloon (3) is equipped with a stent (18) provided with a bevelled end (19).

9. Catheter according to claim 7 or claim 8, characterised in that the portion of the guidewire (9) that comes out from said seat (20) forms the abutment for the catheter (17) positioning onto the saddle (15) of the bifurcation (14), whereby, when the catheter (17) is so positioned, said bevelled end is positioned at the opening of the lateral branch of the bifurcation (14) so not to form projections inside its main blood vessels.

10. Catheter according to one or more of the claims 1 to 3, characterised in that said balloon (3) is provided with at least one opening or seat (22) for the exit in distal position with respect to said balloon of said at least another guidewire (9) in branching or lateral arrangement with respect to the first guidewire (6).

11. Catheter according to claim 10, characterised in that said balloon (3) is equipped with a stent (23) of resistant material.

12. Catheter according to claim 10 or claim 11, characterised in that the portion of the guidewire (9) that comes out from said seat (22) forms the abutment for the catheter (21) positioning onto the saddle (15) of the bifurcation (14), whereby, when the catheter (21) is so positioned, said stent (23) is applied in the main blood vessel of the bifurcation (14).

13. Catheter according to one or more of the preceding claims, characterised in that it has a balloon characterised by a two-chamber structure, at the side exit for the guidewire.

14. Catheter according to one or more of the preceding claims, characterised in that said channels (5, 8) for the access of the respective guidewires to the balloon are separated from each other or in form of a single channel (24) having flattened or ellipsoidal cross-section.

15. Stent for vascular procedures, characterised in that it is composed of a cage (11) made of resistant material having a substantially cylindrical shape and provided with side opening (12).

16. Stent for vascular procedures, characterised in that it is composed of a cage (11) made of resistant material having a substantially cylindrical shape and provided with a bevelled or slant-shaped end (19).

17. Method for applying a catheter in a coronary bifurcation according to one or more of the preceding claims, characterised in that the insertion of said catheter in the blood vessel is carried on until it interferes with the saddle (15) of the bifurcation (14) of the portion of said at least another guidewire (9) that comes out from the respective seat (20, 22, 92) of the balloon (3) in branching or lateral position with respect to the first guidewire (6).

18. Method according to claim 17, characterised in that the insertion of the guidewires automatically produces the insertion of the stent in its correct orientation inside the blood vessels.
